# EUROPÄISCHE PATENTSCHRIFT

(11) **EP 0 416 661 B1**
(45) Veröffentlichungstag und Bekanntmachung des Hinweises auf die Patenterteilung: **30.11.1994**
(21) Anmeldenummer: 90117318.7
(22) Anmeldetag: 07.09.1990
(51) Int. Cl.: C07C 67/58, C07C 69/30, C11B 3/00

(54) **Verfahren zur Herstellung reiner Monoglyceride, reiner Diglyceride und/oder reiner Triglyceride**
Method for the production of pure monoglycerides, pure diglycerides and/or triglycerides
Procédé pour la fabrication de monoglycérides purs, de diglycérides purs et/ou de triglycérides purs

(30) Priorität: 08.09.1989 DE 3930026
(43) Veröffentlichungstag der Anmeldung: 13.03.1991
(73) Patentinhaber: Peter, Siegfried, Prof. Dr., 91080 Uttenreuth-Weiher (DE)
(72) Erfinder: Peter, Siegfried, Prof. Dr., D-8525 Uttenreuth-Weiher (DE); Czech, Peter, Dipl.-Ing., D-8520 Erlangen (DE); Ender, Ulrich, Dipl.-Ing., W-4300 Essen 1 (DE); Weidner, Eckhard, Dr. Dipl.-Ing., D-8520 Erlangen (DE)
(74) Vertreter: Weinhold, Peter, Dr.

(56) Entgegenhaltungen:
- EP-A- 0 269 904
- EP-A- 0 352 667
- DE-A- 2 644 916
- DE-A- 3 825 248
- Soviet Inventions Illustrated, Derwent Publ. Ltd London GB. Sektion Chemie,Woche B25, 01.08.1979 &SU-A-620474 (Mosc. Fats Res. Inst.) (17.07.78)

## Beschreibung

Mono- und Diglyceride sind partielle Ester des Glycerins mit höhermolekularen Fettsäuren. Handelsübliche Monoglyceride bestehen aus Mischungen von Mono- und Diestern mit geringen Anteilen an Triestern. Da die Mono- und Diester des Glycerins eßbar sind, werden sie wegen ihrer emulgierenden, stabilisierenden, plastifizierenden und verdickenden Eigenschaften in verschiedenen Zweigen der Nahrungsmittelindustrie, der Pharmazie und Kosmetik eingesetzt. Für viele, namentlich technische Zwecke kann das bei der Herstellung der Glyceride entstehende Gleichgewichtsgemisch nach Abtrennung des nicht umgesetzten Glycerins unmittelbar verwendet werden. Darüberhinaus ist aber für verschiedene Anwendungsgebiete die Erzeugung von hochprozentigen Monoglyceriden und Diglyceriden erwünscht. So werden durch Molekulardestillation gewonnene Monoglyceride mit einem Monoglyceridgehalt von über 90% hauptsächlich in der Nahrungsmittelindustrie (Teig-, Süß- und Backhilfswaren, Margarine, Speiseeis) verwendet.

Durch Zusatz von Monoglycerid (bis zu 5% Palmitin/Stearinsäure-monoglycerid 90 %-ig oder 10% Palmitin/Stearinsäure-mono/diglycerid) erreicht man selbstemulgierende Eigenschaften der zu Backzwecken bestimmten Backfette (superglycerolated shortenings).

Der Ausdruck "shortening" bedeutet wörtlich verkürzend und geht auf die Backeigenschaften zurück. Aufgrund der besonderen Struktur vermögen die Monoglyceride die plastifizierende Wirkung von Stärke und Gluten bei der Teigbereitung zu verändern, indem sie sich in feiner Verteilung in die homogenen Plastikate einschieben, sie unterbrechen und damit den Teig geschmeidiger machen, d.h. verkürzen. Gleichzeitig wird die Einarbeitung von Luft erleichtert, so daß zusammengenommen Backwaren mit vergrößertem Volumen und besserer "Kürze" entstehen.

Die wesentlichen Bestandteile der Margarine sind Speisefette und -öle, Trinkwasser und Emulgatoren. Als Emulgatoren dienen Lecithin, Eigelb und/oder Mono- und Diglyceride von Speisefettsäuren. Außerdem kann die Margarine noch Geruchs- und Geschmacksstoffe (Aromastoffe), gesäuerte Milch, Magermilch, Speisesalz, Stärkesirup, Zitronensäure und/oder andere Genußsäuren, Vitamine sowie zugelassene Farbstoffe (im allgemeinen Carotin oder carotinhaltige Öle) enthalten. Emulgatoren sind unerläßliche Hilfsstoffe bei der Margarineherstellung, da sie die Bildung von Wasser-in-Öl-Emulsionen ermöglichen. Am gebräuchlichsten sind Monoglyceride und pflanzliches Lecithin, die sich in ihrer emulgierenden Wirkung unterstützen. Zur praktischen Anwendung kommen Produkte mit etwa 40% bzw. etwa 90% Monoglyceriden von C₁₆/C₁₈-Säuren (Palmitinsäure, Stearinsäure, auch im Gemisch mit Ölsäure), die daneben 60% bzw. 10% Diglyceride enthalten. Zusätze von bis zu 0,5% bzw. 0,25%, entsprechend etwa 0,2% Monoglycerid, bezogen auf die Fettphase, sind üblich. Zur Herstellung von Halbfettmargarine benötigt man gewöhnlich höhere Emulgatoranteile.

Mono- und Diglyceride sind durch Verestern von Glycerin mit Fettsäuren zugänglich. Durch Umesterung von Triglyceriden mit Glycerin oder durch Umsetzung von Glycerin mit Fettsäuren in Gegenwart von Katalysatoren entstehen Gemische, die aus Glycerin, Mono-, Di- und Triglyceriden und freien Fettsäuren zusammengesetzt sind. Weiterhin hat die enzymatische Zerlegung von Triglyceriden in neuerer Zeit Eingang in die Technik gefunden. Alle Methoden führen zu einem Gemisch von Mono- Di- und Triglyceriden. Bei der Veresterung stellt sich z.B. ein Gleichgewichtsgemisch ein, das nach Entfernen des Glycerins etwa 60% Mono-, 35% Di- und 5% Triglyceride enthält. Das Ausgangsgemisch bei der Veresterung wird daher so gewählt, daß im Gleichgewicht als Hauptprodukt die Monoglyceride erhalten werden. Das Gemisch wird gewöhnlich durch Molekulardestillation getrennt. Bei den hohen Temperaturen im Filmverdampfer erfolgt in geringem Maße Disproportionierung, so daß Mono- oder Diglyceride kleine Mengen der anderen beiden Ester und geringe Mengen an freien Fettsäuren enthalten. Außerdem wird das Verhältnis von 1-Monoglyceriden zu 2-Monoglyceriden zu Gunsten von 2-Monoglyceriden verschoben.

Wegen der Disproportionierung bei den Temperaturen im Filmverdampfer sind Monoglyceride mit Gehalten über 95% mit der Molekulardestillation wirtschaftlich nicht erhältlich. Es besteht aber Interesse an Monoglyceriden, die eine Reinheit von 99% und darüber besitzen.

Es ist bekannt, daß Monoglyceride aus einem Gemisch von Mono-, Di- und Triglyceriden mit Hilfe von dichtem Kohlendioxid abgetrennt werden können. Dazu sind jedoch Drücke von über 350 atm bei Temperaturen von 40°C erforderlich. Außerdem ist die Beladung selbst bei Drücken von 350 atm noch so gering (weniger als 0,5%), daß eine wirtschaftliche Gewinnung hochprozentiger Monoglyceride nicht möglich ist.

Auch wurde die Verwendung von Aceton als Schleppmittel vorgeschlagen (DE-OS 23 40 566.5). Dabei gelangen die Monoglyceride als die leichter löslichen Komponenten in das Kopfprodukt bei der Gegenstromextraktion. Die Trennfaktoren sind dabei jedoch relativ gering, so daß die Gewinnung reiner Monoglyceride wirtschaftlich nicht interessant ist, zumal die erreichten Beladungen mit 0,5 bis 1,5% gering sind. Außerdem ist die Entfernung des Schleppmittels Aceton aus dem Produkt aufwendig.

Weiterhin wurde die Verwendung von Kohlenwasserstoffen als Kosolvens zu einem überkritischen Extraktionsmittel wie Kohlendioxid, N₂O, Schwefelhexafluorid, Trifluormethan oder Tetrafluormethan vorgeschlagen (deutsche Patentanmeldung P 38 25 248.1). Dieses Verfahren hat die oben genannten Nachteile nicht. Jedoch ist die Verteilung von Kosolvens (Schleppmittel) und überkritischer Komponente zwischen fluider und flüssiger Phase nicht gleichmäßig. Dadurch werden beim Ausschleusen der Produkte unterschiedliche Mengen an Kosolvens und überkritischer Komponente aus dem Extraktionsmittelkreislauf ausgeschleust. Deswegen muß mit Hilfe einer etwas aufwendigen Regel- und Analysenvorrichtung die Zusammensetzung des Extraktionsmittels laufend korrigiert werden.

Aufgabe der Erfindung ist die Schaffung eines Verfahrens zur Gewinnung reiner Mono- und reiner Diglyceride durch einen vereinfachten und wirtschaftlicheren Verfahrensablauf.

Dies konnte nun überraschenderweise mit dem Verfahren der vorliegenden Erfindung erzielt werden.

Erfindungsgemäß wird nun ein Verfahren zur Gewinnung von Monoglyceriden und ggf. Diglyceriden und/oder Triglyceriden sowie ggf. Glycerin aus Glyceridgemischen bzw. glycerinhaltigen Glyceridgemischen durch Gegenstromextraktion mit einem im Kreislauf gehaltenen Extraktionsmittel vorgeschlagen, welches dadurch gekennzeichnet ist, daß ein Kohlenwasserstoff mit einer Dichte von mehr als 180 kg/m³ und/oder Trifluormethan mit einer Dichte von mehr als 180 kg/m³ als eine separate Phase bildendes Extraktionsmittel verwendet wird.

Bei dem erfindungsgemäßen Verfahren werden Temperatur und Druck so gewählt, daß das System aus dem Glyceridgemisch oder glycerinhaltigen Glyceridgemisch und dem Extraktionsmittel in der Trennsäule und in den Fraktionierkolonnen zweiphasig ist. Das ist in einem Dichtebereich der leichtflüchtigen Kohlenwasserstoffe mit 2 bis 5 Kohlenwasserstoffatomen von 180 bis 800 kg/m³ möglich. Bei höheren Dichten wird das System aus Glyceridgemisch und Kohlenwasserstoff einphasig, so daß eine Trennoperation unmöglich ist. Bei geringeren Dichten ist die Beladung des Extraktionsmittels für wirtschaftliches Arbeiten zu gering.

Es wurde überraschenderweise gefunden, daß in dem angewendeten Dichtebereich Arbeitsbedingungen angewandt werden können, bei denen einerseits das System aus Glyceridgemisch und leicht flüchtigem Kohlenwasserstoff zweiphasig ist und andererseits die Beladung des Extraktionsmittels bei ausreichenden Trennfaktoren wirtschaftliches Arbeiten möglich macht. Bei dem erfindungsgemäßen Verfahren ist der Zusatz einer überkritischen Komponente mit schlechtem Lösevermögen wie z.B. CO₂ zur Erzeugung von Zweiphasigkeit nicht erforderlich.

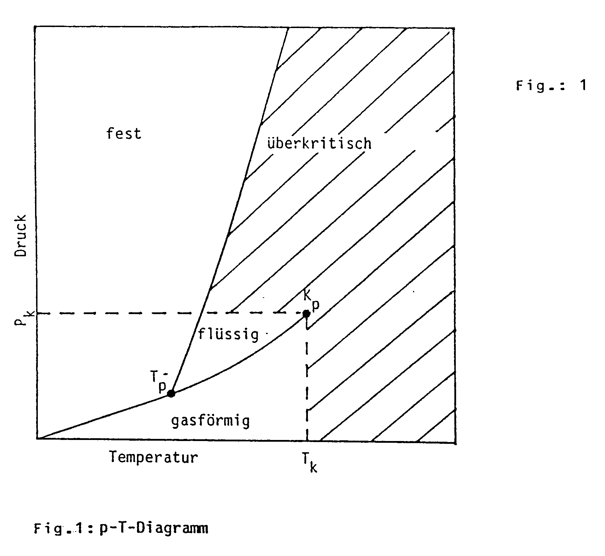

Nach dem Verfahren dieser Erfindung sind durch die Verwendung von niedermolekularen Kohlenwasserstoffen, wie z.B. Ethan, Propan, Butan, Pentan, Ethen, Propen, Buten sowie Trifluormethan oder deren Gemische als überkritische Extraktionsmittel hohe Trennfaktoren in Verbindung mit relativ hoher Beladung realisierbar. Dabei sollen Substanzen als überkritisch bezeichnet werden, deren Temperatur bei entsprechenden Drücken höher als die kritische Temperatur bzw. deren Druck bei der entsprechenden Temperatur höher als der kritische Druck ist (schraffiertes Feld, gemäß Fig. 1). Bevorzugtes Arbeiten findet im sogenannten "Near-critical"-Zustand statt. Unter dem "Near-critical"-Zustand werden Arbeitsbedingungen verstanden, bei denen Druck und Temperatur so gewählt werden, daß sie nahe der Grenzen des schraffierten Bereichs in Fig. 1 liegen. So kann z.B. auch bei einer Temperatur unterhalb der kritischen Temperatur gearbeitet werden, wenn der entsprechende Druck überhalb des kritischen Drucks gewählt wird.

Der bevorzugte Dichtebereich bei der erfindungsgemäßen Arbeitsweise liegt für Kohlenwasserstoffe bei 200 bis 800 kg/m³ und für Trifluormethan bei 300 bis 1100 kg/m³.

Die Trennfaktoren zwischen Monoglyceriden und Diglyceriden und zwischen Diglyceriden und Triglyceriden sind im genannten Dichtebereich so groß, daß eine Zerlegung des Glyceridgemisches in Monoglyceride, Diglyceride und Triglyceride in einem Arbeitsgang möglich ist. Ebenso ist in diesem Dichtebereich die Zerlegung eines glycerinhaltigen Glyceridgemisches in Glycerin, Mono-, Di- und Triglyceride möglich.

Ein weiterer wesentlicher Vorteil des neuen Verfahrens ist die geringe thermische Belastung der Produkte. Die Trennung z.B. der Glyceride der C₁₈- und der C₁₆-Säuren mit Hilfe der Molekulardestillation erfordert im Verdampfer Temperaturen von etwa 200°C. Dagegen sind die Temperaturen bei der "near critical fluid extraction" durch die Schmelztemperaturen der Glyceride gegeben. So schmelzen z.B. die Glyceride der Stearinsäure zwischen 70 und 80°C. Demnach kann bei der Trennung von Stearinsäureglyceriden bei Temperaturen von 90 bis 110°C gearbeitet werden. Die Schmelzpunkte der Ölsäureglyceride liegen um 30°. In diesem Fall wird man deshalb Temperaturen von 40 bis 60°C für die Trennung vorziehen. Durch die erforderlichen niedrigen Temperaturen des erfindungsgemäßen Verfahrens ist auch die Gefahr der Disproportionierung der Glyceride, die bei der Molekulardestillation gegeben ist, wesentlich reduziert, wodurch die Herstellung von Produkten mit einem Monoglyceridgehalt von 99 bis 99,5% ermöglicht wird.

Wird beispielsweise Ethan als Extraktionsmittel verwendet, so ergeben sich geeignete Arbeitsbedingungen für die Trennung von Glyceridgemischen oder glycerinhaltigen Glyceridgemischen bei Temperaturen von 10 bis 120°C, vorzugsweise von 20 bis 80°C und Drücken von 50 bis 500 bar, vorzugsweise von 100 bis 350 bar.

Die Dichte von Ethan beträgt z.B. bei 20°C und einem Druck von 300 atm 450 kg/m³. Die Beladung des Ethans im Gleichgewicht mit einem Glyceridgemisch aus 59 Gew.% Monoglyceriden, 36 Gew.% Diglyceriden, 4,6 Gew.% Triglyceriden der Ölsäure und 0,4 Gew.% freien Fettsäuren beträgt unter den genannten Bedingungen 1 Gew.%. Durch Erhöhen der Temperatur auf etwa 100°C und Verminderung des Druckes auf etwa 50 atm kann die Beladung bei der Regenerierung des Extraktionsmittels auf Werte von weniger als 0,02 Gew.% vermindert werden.

Temperaturen von 40 bis 150°C, vorzugsweise von 60 bis 120°C und Drücken von 40 bis 350 bar, vorzugsweise von 70 bis 250 bar sind zweckmäßig, wenn Propan als Extraktionsmittel verwendet wird.

Die Dichte des Propans beträgt z.B. bei 110°C und einem Druck von 85 bar 360 kg/m³. Die Beladung des Propans im Gleichgewicht mit einem Glyceridgemisch aus 61 Gew.% Monoglyceriden, 35 Gew.% Diglyceriden, 3,5 Gew.% Triglyceriden der Stearinsäure und 0,5 Gew.% freien Fettsäuren liegt unter diesen Bedingungen bei 5,4 Gew.%. Durch Erhöhen der Temperatur auf etwa 120°C und Verminderung des Druckes auf etwa 35 bar kann die Beladung bei der Regenerierung des Extraktionsmittels auf Werte von weniger als 0,04 Gew.% vermindert werden.

Aber auch unterhalb der kritischen Temperatur z.B. des Propans bei 80°C ist die Trennung der Monoglyceride aus einem Glyceridgemisch möglich. Bei 80°C und einem Druck von 60 bar beträgt die Dichte des Propans 410 kg/m³. Die Beladung des Propans im Gleichgewicht mit einem Glyceridgemisch aus 61 Gew.% Monoglyceriden, 35 Gew.% Diglyceriden, 3,5 Gew.% Triglyceriden der Stearinsäure und 0,5 Gew.% freien Fettsäuren liegt unter diesen Bedingungen bei 6 Gew.%. Durch Erhöhen der Temperatur auf etwa 110°C und Verminderung des Drucks auf etwa 30 bar kann die Beladung auf Werte von weniger als 0,02 Gew.% herabgesetzt werden. Die Dichte des Propans beträgt unter diesen Bedingungen 57 kg/m³.

Allgemein kann gesagt werden, daß zur Regenerierung des Extraktionsmittels dessen Dichte vorzugsweise auf 1/5 bis 1/7 der ursprünglichen Dichte vermindert wird.

Nach dem Verfahren dieser Erfindung erscheinen im Gegensatz zu dem in der DE-OS 23 40 566.5 beschrieben Verfahren die Monoglyceride nicht als Kopfprodukt sondern als Sumpfprodukt in der Gegenstromkolonne, im folgenden auch als Trennsäule oder Trennkolonne bezeichnet. Als Extrakt enthält das Extraktionsmittel am Kopf der Kolonne die Di- und Triglyceride, die nachfolgend durch fraktionierte Ausfällung aus dem Extraktionsmittel abgetrennt werden können. Zur fraktionierten Ausfällung können zwei Fraktionierkolonnen verwendet werden. Wenn auf fraktionierte Abscheidung der Di- und Triglyceride verzichtet wird, genügt eine Fraktionierkolonne. In der Trennsäule und in den Fraktionierkolonnen liegen zwei Phasen vor. Zwischen Kopf der Trennsäule und Entspannungsventil ist der zirkulierende Extrakt einphasig. Bei der Entspannung fallen die gelösten Komponenten als flüssige Phase aus. In den Fraktionierkolonnen wird die entstandene flüssige Phase abgetrennt. Danach ist das zirkulierende Extraktionsmittel einphasig.

In einer Ausführungsform des erfindungsgemäßen Verfahrens, bei dem in einer Gegenstromkolonne das Extraktionsmittel von unten nach oben strömt und das zu trennende Glyceridgemisch in der Mitte oder am Kopf zugegeben wird, fließt im Gegenstrom die flüssige Phase nach unten. Letztere verarmt auf dem Weg nach unten an Di- und Triglyceriden, bis schließlich ein Sumpfprodukt mit einem Monoglyceridgehalt von über 99% anfällt. Das am Kopf dieser Kolonne austretende Extraktionsmittel enthält neben einem geringfügigen Rest an Monoglyceriden die im Zulauf vorhandenen Anteile an Di- und Triglyceriden. Nach entsprechender Verminderung der Dichte z.B. durch Druckverminderung und/oder Temperaturerhöhung wird das die Trennkolonne verlassende Extraktionsmittel der anschließenden Kolonne, im folgenden auch als Fraktionierkolonne bezeichnet, etwa in der Mitte zugegeben. Durch die Dichteverminderung fallen in der Fraktionierkolonne bevorzugt die Diglyceride aus, die als flüssige Phase nach unten fließen. Auf dem Weg nach unten verarmt die ausgefällte Flüssigkeit an Triglyceriden, bis schließlich ein Sumpfprodukt mit hohem Gehalt an Diglyceriden anfällt. Ein Teil des in der Fraktionierkolonne angefallenen Sumpfproduktes kann als Rücklauf auf die Trennkolonne gegeben und der Rest als Produkt, welches Diglyceride in großer Reinheit enthält, entnommen werden.

Das am Kopf der Fraktionierkolonne austretende Extraktionsmittel enthält neben einem geringfügigen Rest an Diglyceriden die im Zulauf vorhandenen Anteile an Triglyceriden. Nach weiterer Verminderung der Dichte wird das die Fraktionierkolonne verlassende Extraktionsmittel der dritten Kolonne, die auch als Regenerierkolonne bezeichnet wird, im mittleren Teil zugeführt. Infolge der Dichteverminderung fallen in der Regenerierkolonne die noch im Extraktionsmittel gelösten schwerflüchtigen Stoffe praktisch vollständig aus und fließen als flüssige Phase nach unten. Ein Teil der in der Regenerierkolonne ausgefällten Glyceride kann als Rücklauf auf die Fraktionierkolonne zurückgeführt und der Rest als Produkt, das hauptsächlich Triglyceride enthält, entnommen werden.

Ein Teil des Kreisgases, d.h. des in der Regenerierkolonne regenerierten Extraktionsmittels, wird nach entsprechender Dichteerhöhung z.B. (durch Druckerhöhung und/oder Temperaturverminderung am Boden der Trennkolonne wieder zugegeben und strömt von unten nach oben. Ebenso kann ein Teil des regenerierten Extraktionsmittels nach entsprechender Dichteerhöhung am Boden der Fraktionierkolonne zugeführt werden.

Auf diese Weise gelingt es nach dem Verfahren dieser Erfindung die Auftrennung des Gemisches aus Mono-, Di- und Triglyceriden in drei Fraktionen, die jeweils die Monoglyceride, die Diglyceride und die Triglyceride in hoher Reinheit enthalten, in einem Arbeitsgang, ohne daß dabei eine aufwendige Regel- und Analyseneinrichtung verwendet werden muß, die laufend die Zusammensetzung des Extraktionsmittels korrigiert.

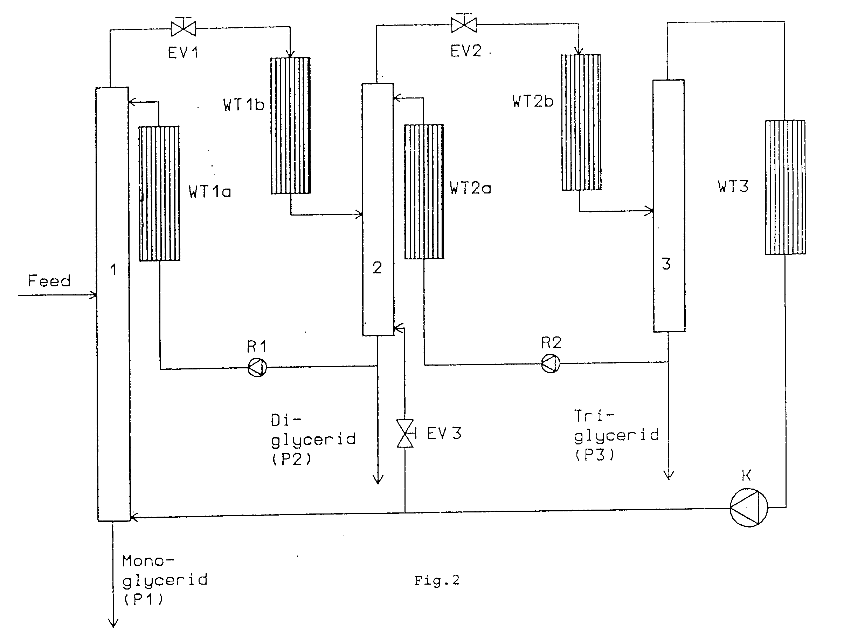

Anhand der schematischen Darstellung in Fig. 2 sei eine Ausführungsform des erfindungsgemäßen Verfahrens näher erläutert: Die Apparatur besteht aus drei Kolonnen, von denen die eine, die Trennkolonne 1 zur Abtrennung der Monoglyceride aus dem Glyceridgemisch dient. Die Monoglyceride werden als Sumpfprodukt erhalten. Die zweite Kolonne dient als Fraktionierkolonne zur Abtrennung der Diglyceride in hoher Reinheit. Die dritte Kolonne, die Regenerierkolonne, dient zur Abtrennung der restlichen extrahierten Komponenten aus dem Kreisgas. Das Glyceridgemisch, das zuvor von Glycerin befreit wurde, wird im mittleren Teil der Trennkolonne zugeführt. Das zirkulierende Extraktionsmittel belädt sich in der Trennkolonne bevorzugt mit Di- und Triglyceriden. Gleichzeitig löst sich das Extraktionsmittel in der sich mit Monoglyceriden anreichernden nach unten strömenden Flüssigphase auf.

Das mit den leichter löslichen Komponenten, den Di- und Triglyceriden, beladene Extraktionsmittel verläßt den Verstärkerteil der Trennkolonne 1 am Kopf. Das beladene Extraktionsmittel wird über das Druckreduzierventil EV1 entspannt und im mittleren Teil der Fraktionierkolonne 2 nach Durchlaufen des Wärmetauschers WT1b zugeführt. Zur Dichteänderung werden Druck und Temperatur in der Fraktionierkolonne 2 so gewählt, daß bevorzugt Diglyceride und der geringe Rest an Monoglyceriden abgeschieden werden.

Die kondensierte Phase wird aus dem Sumpf der Fraktionierkolonne abgezogen und in Produkt P2 und Rücklauf R1 geteilt. Der Rücklauf wird nach Durchlaufen des Wärmetauschers WT1a am Kopf der Trennkolonne 1 aufgegeben.

Der mit vorwiegend Triglyceriden beladene Extraktionsmittelstrom aus der Fraktionierkolonne 2 wird über das Druckreduzierventil EV2 und nach Durchlaufen des Wärmetauschers WT2b in die Regenerierkolonne 3 entspannt. In der Regenerierkolonne 3 erfolgt eine durch Druck- und/oder Temperaturänderung bedingte Dichteverminderung, so daß das die Kolonne verlassende Extraktionsmittel frei von schwerflüchtigen Komponenten ist. Die kondensierte Phase, die neben Triglyceriden noch freie Fettsäuren und geringe Mengen an Diglyceriden enthält, wird aus dem Sumpf der Regenerierkolonne 3 abgezogen und in Produkt P3 und Rücklauf R2 geteilt. Der Rücklauf aus Regenerierkolonne 3 wird nach Durchlaufen des Wärmetauschers WT2a am Kopf der Fraktionierkolonne 2 aufgegeben.

Das regenerierte Extraktionsmittel verläßt die Regenerierkolonne 3 am Kopf und wird mit Hilfe eines Kompressors K nach Durchlaufen des Wärmetauschers WT3 der Trennkolonne 1 wieder zugeführt. Ein kleinerer Teil des regenerierten Kreisgases wird über das Druckreduzierventil EV3 teilentspannt und am Boden der Fraktionierkolonne 2 zugeführt.

Aus der Fraktionierkolonne 2 werden hochprozentige Diglyceride als Sumpfprodukt und aus der Regenerierkolonne 3 ein triglyceridreiches Sumpfprodukt erhalten. Die anfallenden drei verschiedenen Sumpfprodukte werden kontinuierlich abgezogen und in Behälter entspannt. Die sich dabei entlösenden Mengen an gasförmigem Extraktionsmittel werden über einen Kompressor in den Kreislauf, zurückgegeben.

Bei Fraktionierung der im Extraktionsmittel gelösten Komponenten wird bei isothermer Fahrweise der Druck in der Fraktionierkolonne 2 um etwa 5 bis 60 bar, vorzugsweise um 15 bis 40 bar, gegenüber dem Druck in der Trennkolonne 1 vermindert. Die Fraktionierung kann auch isobar erfolgen. In diesem Fall wird die Temperatur in der Fraktionierkolonne 2 um 10 bis 80°C, vorzugsweise von 20 bis 50°C, gegenüber der Temperatur in der Trennkolonne 1 erhöht. Eine Kombination von Verminderung des Druckes und Erhöhung der Temperatur ist ebenfalls möglich. Die Regenerierung des Extraktionsmittels erfolgt entweder durch Entspannung allein oder durch Entspannung bei gleichzeitiger Temperaturerhöhung. Der Druck bei der Regenerierung beträgt 25 bis 80 bar im Temperaturbereich von 40 bis 120°C.

Um die Entstehung der Einphasigkeit in der Trennkolonne infolge abnehmenden Gehaltes an Monoglyceriden im Verstärkerteil der Trennkolonne 1 zu verhindern, ist es zweckmäßig, die Temperatur zum Kolonnenkopf hin zu erhöhen. Je nach der Zusammensetzung des Glyceridgemisches in Hinsicht auf die beteiligten Fettsäuren ist eine Temperaturdifferenz zwischen Kopf und Sumpf der Kolonne von 5 bis 30°C, vorzugsweise von 10 bis 20°C, vorteilhaft. Ebenso ist es zweckmäßig, ein Temperaturgefälle zwischen Kopf und Sumpf der Fraktionierkolonne 2 vorzusehen. In diesem Fall beträgt die Temperaturdifferenz zwischen Kopf und Sumpf 5 bis 40°C, vorzugsweise 10 bis 30°C.

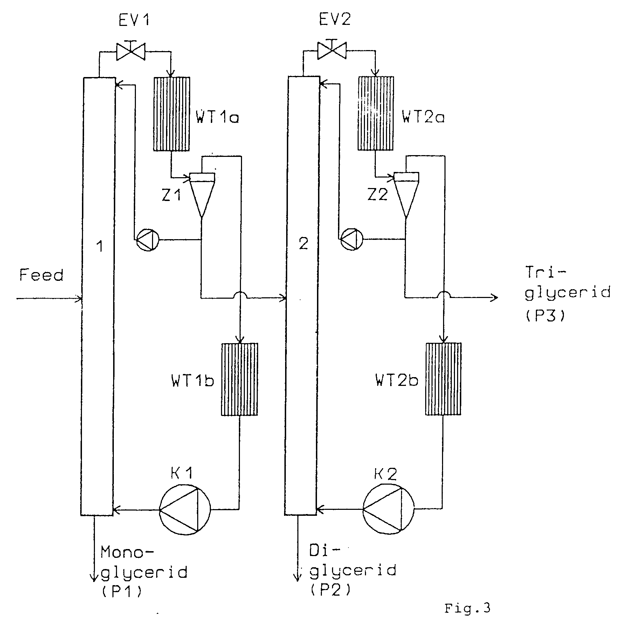

Eine weitere Ausführungsform des erfindungsgemäßen Verfahrens ist anhand der schematischen Darstellung in Fig. 3 dargestellt: Die Apparatur besteht aus zwei Gegenstromkolonnen mit separaten Kreisläufen, die in einer Kaskade hintereinander geschaltet sind. Der glycerinhaltige Zulauf des Glyceridgemisches wird der ersten Kolonne 1 im mittleren Teil zugeführt. Die Betriebsbedingungen werden so gewählt, daß das zirkulierende Extraktionsmittel bevorzugt die Di- und Triglyceride löst. Gleichzeitig löst sich das Extraktionsmittel in der sich mit Glycerin und Monoglyceriden anreichernden nach unten fließenden Flüssigkeit auf. Als Sumpfprodukt wird ein Gemisch aus Glycerin und Monoglyceriden erhalten. Das Glycerin kann von den Monoglyceriden durch Wasserwäsche oder Vakuumdestillation entfernt werden.

Das mit Di- und Triglyceriden beladene Extraktionsmittel wird im Druckreduzierventil EV1 entspannt und im Wärmetauscher WT1a erwärmt. Dabei fallen die gelösten Di- und Triglyceride aus. Sie werden im Zyklon Z1 aus dem Extraktionsmittelstrom abgetrennt. Das von Flüssigkeitströpfchen befreite Extraktionsmittel wird nach Durchlaufen des Wärmetauschers WT1b mit Hilfe des Kreislaufkompressors K1 oberhalb des Sumpfes in die Kolonne 1 zurückgeführt. Ein Teil des im Zyklon Z1 abgetrennten Produktes wird als Rücklauf auf den Kopf der Kolonne 1 zurückgegeben. Der Rest wird in die Kolonne 2 übergeführt.

In der Kolonne 2 werden Druck und Temperatur so gewählt, daß die Triglyceride als Kopfprodukt abgetrennt werden. Das mit Triglycerid beladene Extraktionsmittel verläßt die Kolonne 2 am Kopf, wird im Druckreduzierventil EV2 entspannt und im Wärmetauscher WT2a erwärmt. Dabei fallen die im Extraktionsmittel gelösten Triglyceride aus. Im Zyklon Z2 werden die ausgefallenen Triglyceride hoher Reinheit vom Extraktionsmittel abgetrennt. Das regenerierte Extraktionsmittel wird nach Durchlaufen des Wärmetauschers WT2b mit Hilfe des Kreisgaskompressors K2 oberhalb des Sumpfes in Kolonne 2 rezirkuliert. Die im Zyklon abgeschiedene Flüssigkeit wird in den Rücklauf der Kolonne 2 und das Produkt P3 geteilt. Der Rücklauf wird auf die Kolonne 2 zurückgegeben. Das Sumpfprodukt der Kolonne 2 besteht aus Diglyceriden, welche in großer Reinheit gewonnen werden.

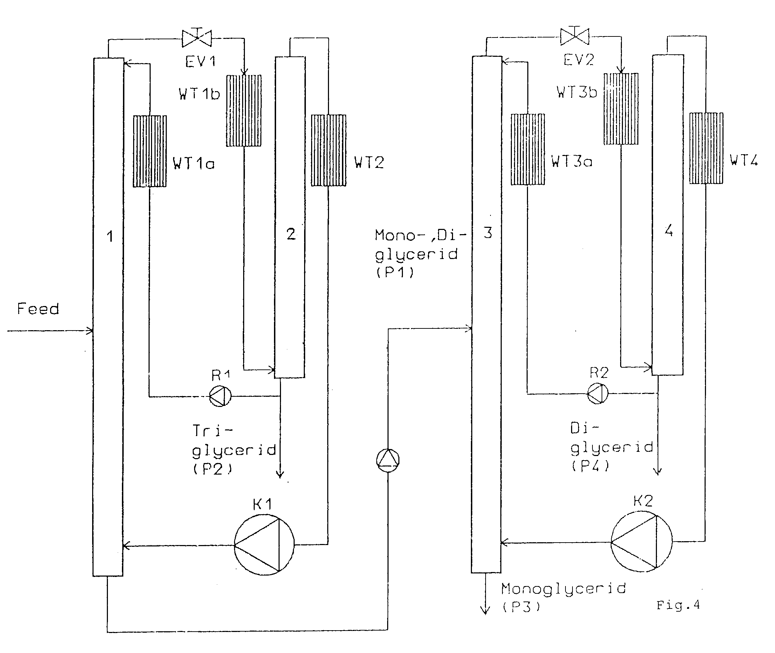

Eine weitere Ausführungsform des erfindungsgemäßen Verfahrens ist in Fig. 4 gezeigt. Die Anlage besteht aus der Trennkolonne 1, der Regenerierkolonne 2, der weiteren Trennkolonne 3 und der Regenerierkolonne 4. Der zuvor durch Wasserwäsche oder Destillation von Glycerin befreite Feed wird der Kolonne 1 in der Mitte zugeführt. Druck und Temperatur werden so gewählt, daß die Triglyceride mit dem Extraktionsmittel am Kopf der Säule abgeführt werden. Das mit den Triglyceriden beladene Extraktionsmittel verläßt die Kolonne 1 am Kopf, wird im Druckreduzierventil EV1 entspannt und im Wärmetauscher WT1b erwärmt. Dabei fallen die im Extraktionsmittel gelösten Triglyceride aus und werden in der Regenerierkolonne 2 abgeschieden. Die als Sumpfprodukt P2 anfallenden Triglyceride werden abgezogen und ein Teil derselben als Rücklauf R1 nach Durchlaufen des Wärmetauschers WT1a auf den Kopf der Trennkolonne 1 zurückgeführt. Das die Regenerierkolonne 2 verlassende Kreisgas passiert den Wärmetauscher WT2 und wird oberhalb des Sumpfes in die Trennkolonne 1 mit Hilfe des Kompressors K1 gegeben.

Das Sumpfprodukt P1 aus Kolonne 1 enthält Mono- und Diglyceride und wird der Trennkolonne 3 mit Hilfe eines Kompressors als Feed zugeführt. In der Trennkolonne 3 werden Druck und Temperatur so gewählt, daß sich die Diglyceride bevorzugt im zirkulierenden Extraktionsmittel lösen. Gleichzeitig löst sich das Extraktionsmittel in der sich mit den Monoglyceriden anreichernden nach unten fließenden Flüssigphase. Das mit den leichter löslichen Diglyceriden beladene Extraktionsmittel verläßt den Verstärkerteil der Gegenstromkolonne 3 am Kopf. Das beladene Extraktionsmittel wird über das Druckreduzierventil EV2 entspannt und im Wärmetauscher WT3b erwärmt. Dabei fallen die gelösten Diglyceride als Tröpfchen aus. In der Regenerierkolonne 4 wird die kondensierte Phase vom Extraktionsmittel abgetrennt. Ein Teil der kondensierten Phase wird nach Durchlaufen des Wärmetauschers WT3a auf den Kopf der Kolonne 3 als Rücklauf R2 zurückgeführt und der Rest als Produkt P4 entnommen. Das regenerierte Kreisgas verlaßt die Regenerierkolonne 4 am Kopf und wird nach Passieren des Wärmetauschers WT4 mit Hilfe des Kreislaufkompressors K2 in die Kolonne 3 oberhalb des Sumpfes zurückgegeben. Die Monoglyceride fallen in der Kolonne 3 als Sumpfprodukt P3 an.

Die angefallenen drei verschiedenen Produkte P2, P3, P4 werden kontinuierlich abgezogen und in Behälter auf Atmosphärendruck entspannt. Die sich dabei entlösenden Mengen an gasförmigen Extraktionsmittel werden über einen Kompressor in die Kreisläufe zurückgegeben.

Bei isothermer Fahrweise wird der Druck in Kolonne 3 um etwa 5 bis 60 bar, vorzugsweise um 15 bis 40 bar, gegenüber dem Druck in Kolonne 1 erhöht. Bei isobarer Fahrweise wird die Temperatur in der Kolonne 3 um 10 bis 80°C, vorzugsweise um 20 bis 50°C, gegenüber der Temperatur in der Kolonne 1 vermindert, d.h. gegenüber Kolonne 1 erfolgt für Kolonne 3 eine Dichteerhöhung des Extraktionsmittels. Dies kann jedoch auch durch Kombination von Verminderung der Temperatur und Erhöhung des Druckes erzielt werden.

Um die Entstehung von Einphasigkeit infolge abnehmenden Gehaltes der schwerlöslichen Komponenten in den Verstärkerteilen der Kolonnen zu verhindern, ist es zweckmäßig, die Temperatur im Kolonnenkopf gegenüber der Temperatur im Sumpf zu erhöhen. Je nach der Zusammensetzung des Glyceridgemisches, bezogen auf die beteiligten Fettsäuren, ist eine Temperaturdifferenz zwischen Kopf und Sumpf der Kolonne von 5 bis 40°C, vorzugsweise von 10 bis 30°C, vorteilhaft.

In den oben beschriebenen Ausführungsformen wird das Glyceridgemisch bzw. glycerinhaltige Glyceridgemisch bevorzugt in der Mitte der verschiedenen Kolonnen zugeführt. Prinzipiell ist es aber auch möglich, das Gemisch am Kopf der entspechenden Säulen oder bei einigen Ausführungsformen auch in einem Bereich zwischen Sumpf und Mitte der Kolonne aufzugeben.

Das erfindungsgemäße Verfahren kann auch mit Hilfe von Druckpulsation durchgeführt werden. Die Pulsationsvorrichtung wird hierbei vorzugsweise oberhalb des Sumpfes der Trennkolonne angebracht. Sie ist als Kolbenmaschine mit Geradschubkurbeltrieb ausgeführt. Ein Verhältnis von verdrängtem Volumen zum Kolonnenvolumen zwischen 1:40 und 1:200 ist für das Verfahren nach dieser Erfindung günstig. Neben einer variablen Hubverstellung wird die Pulsationsvorrichtung mit einer Frequenzregelung ausgestattet, um sich den Bedingungen optimal anpassen zu können. Die im Fluid-Pulser erzeugte Volumenverdrängung ist sinusförmig.

Für die Ausrüstung der Kolonnen sind im Prinzip die bei der Flüssig-Flüssig-Extraktion und der Rektifikationstechnik üblichen Packungen wie Raschigringe, Drahtgewebepackungen, Berlsättel, Drahtwendeln usw. geeignet. Gute Ergebnisse werden besonders mit geordneten Drahtgewebepackungen wie beispielsweise Sulzer CY erhalten. Eine Ausrüstung der Kolonnen mit Siebböden ist ebenfalls möglich.

### Beispiel 1

Ein Glyceridgemisch aus 59 Gew.% Monoglyceriden, 36 Gew.% Diglyceriden, 4,6 Gew.% Triglyceriden der Ölsäure und 0,4 Gew.% freien Fettsäuren wurde bei einer Temperatur von 20°C und einem Druck von 300 bar (bei einer Dichte von 450 kg/m³) einer Gegenstromkolonne am Kopf zugepumpt und mit reinem Ethan als Extraktionsmittel extrahiert. Die Kolonne war 6 m hoch und mit einer Sulzer-Drahtgewebepackung CY ausgerüstet. Die Beladung des Extraktionsmittels bei Verlassen der Kolonne am Kopf betrug 0,66 Gew.%.

Der die Trennkolonne verlassende Extraktstrom wurde in den mittleren Teil der Regenerierkolonne übergeführt. Die Regenerierbedingungen waren 54 bar und 102°C (die Dichte des Ethans beträgt unter diesen Bedingungen 66 kg/m³). Infolge der Druckverminderung und gleichzeitigen Temperaturerhöhung nahm die Beladung des Extraktionsmittels auf 0,02 Gew.% ab. Das regenerierte Extraktionsmittel wurde auf 20°C gekühlt und mit Hilfe eines Umlaufkompressors in die Trennkolonne oberhalb des Sumpfes zurückgeführt.

Das erhaltene Sumpfprodukt enthielt 91 Gew.% Monoglyceride, 8,5 Gew.% Diglyceride und 0,5 Gew.% freie Fettsäuren. Die Konzentration der Triglyceride lag unterhalb der Nachweisgrenze von 0,1 Gew.%. Das in der Regenerierkolonne anfallende Produkt enthielt 21,3 Gew.% Monoglyceride, 68 Gew.% Diglyceride, 10 Gew.% Triglyceride und 0,7 Gew.% freie Fettsäuren.

### Beispiel 2

Ein Glyceridgemisch aus 61 Gew.% Monoglyceriden, 35 Gew.% Diglyceriden, 3,5 Gew.% Triglyceriden der Stearinsäure und 0,5 Gew.% freien Fettsäuren wurde bei einer Temperatur von 110°C und einem Druck von 85 bar einer Gegenstromkolonne am Kopf zugepumpt und mit Propan als Extraktionsmittel extrahiert. Die Dichte des Propans beträgt dabei 366 kg/m³. Die Kolonne war 6 m hoch und mit einer Sulzer-Drahtgewebepackung CY ausgerüstet. Die Beladung des die Kolonne am Kopf verlassenden Extraktionsmittels betrug 3,8 Gew.%. Der die Trennkolonne am Kopf verlassende Extraktstrom wurde der Regenerierkolonne im mittleren Teil zugeführt. Die Regenerierbedingungen waren 35 bar und 120°C. Unter diesen Bedingungen beträgt (die Dichte des Propans 66 kg/m³. Infolge der Druckverminderung und gleichzeitigen Temperaturerhöhung nahm die Beladung des Extraktionsmittels auf 0,04 Gew.% ab. Das regenerierte Extraktionsmittel wurde auf 100°C gekühlt und mit Hilfe eines Umlaufkompressors in die Extraktionskolonne oberhalb des Sumpfes zurückgeführt.

Das erhaltene Sumpfprodukt der Trennkolonne enthielt 99 Gew.% Monoglyceride, 0,7 Gew.% Diglyceride und 0,3 Gew.% freie Fettsäuren. Die Konzentration der Triglyceride lag unterhalb der Nachweisgrenze von 0,1 Gew.%. Das in der Regenerierkolonne anfallende Produkt enthielt 31,5 Gew.% Monoglyceride, 61,6 Gew.% Diglyceride, 6,2 Gew.% Triglyceride und 0,7 Gew.% freie Fettsäuren.

### Beispiel 3

Ein Glyceridgemisch aus 50 Gew.% Monoglyceriden, 37,5 Gew.% Diglyceriden, 11 Gew.% Triglyceriden der Stearinsäure und 1,5 Gew.% freien Fettsäuren wurde bei einer Temperatur von 100°C und einem Druck von 140 bar einer Gegenstromkolonne am Kopf zugepumpt und mit einem Gemisch aus Ethan und Propan extrahiert. Das Extraktionsmittel mit einem Propangehalt von 60 Gew.% ist unter diesen Bedingungen überkritisch, d.h. einphasig. Seine Dichte beträgt 354 kg/m³. Die Kolonne war 12 m hoch und mit einer Sulzer-Drahtgewebepackung CY ausgerüstet. Die Beladung des Extraktionsmittels beim Verlassen der Kolonne am Kopf betrug 1,2 Gew.%.

Der die Trennkolonne am Kopf verlassende Extraktstrom wurde der Regenerierkolonne im mittleren Teil zugeführt und dabei auf 40 bar entspannt und auf 120°C aufgeheizt. Dadurch wurde die Beladung des Extraktionsmittels mit Glyceriden und freien Fettsäuren auf 0,03 Gew.% vermindert. Die Dichte des Extraktionsmittels beträgt unter diesen Bedingungen 57 kg/m³.

Das regenerierte Extraktionsmittel wurde auf 100°C gekühlt und mit Hilfe eines Umlaufkompressors der Gegenstromkolonne oberhalb des Sumpfes zugeführt.

Das am Sumpf der Extraktionskolonne abgezogene Produkt enthielt 85 Gew.% Monoglyceride, 12 Gew.% Diglyceride, 2 Gew.% Triglyceride und 1 Gew.% freie Fettsäuren. Das bei der Regenerierung abgeschiedene Produkt enthielt 35 Gew.% Monoglyceride, 48 Gew.% Diglyceride, 15 Gew.% Triglyceride und 2 Gew.% freie Fettsäuren.

### Beispiel 4

Ein Glyceridgemisch aus 59 Gew.% Monoglyceriden, 36 Gew.% Diglyceriden, 4,3 Gew.% Triglyceriden der Ölsäure und 0,7 Gew.% freien Fettsäuren wurde bei einer Temperatur von 60°C und einem Druck von 80 bar einer Gegenstromkolonne am Kopf zugepumpt und mit Propan extrahiert. Seine Dichte betrug unter diesen Bedingungen 465 kg/m³.

Die Kolonne war 6 m hoch und mit einer Sulzer-Drahtgewebepackung CY ausgerüstet. Die Beladung des Extraktionsmittels mit Schwerflüchtigem betrug 5,7 Gew.%.

Der die Trennkolonne am Kopf verlassende Extraktstrom wurde der Regenerierkolonne im mittleren Teil zugeführt. Die Arbeitsbedingungen bei der Regenerierung waren 35 bar und 100°C (Propandichte 83 kg/m³). Infolge der Druckverminderung und der gleichzeitigen Temperaturerhöhung nahm die Beladung des Extraktionsmittels auf 0,06 Gew.% ab. Das regenerierte Extraktionsmittel wurde auf 60°C gekühlt und mit Hilfe eines Umlaufkompressors in die Extraktionskolonne oberhalb des Sumpfes zurückgeführt.

Das Sumpfprodukt der Trennkolonne enthielt 99,5 Gew.% Monoglyceride, 0,3 Gew.% Diglyceride und 0,2 Gew.% freie Fettsäuren. Die Konzentration der Triglyceride lag unterhalb der Nachweisgrenze von 0,1 Gew.%. Das in der Regenerierkolonne anfallende Produkt enthielt 9,5 Gew.% Monoglyceride, 80,5 Gew.% Diglyceride, 9 Gew.% Triglyceride und 1 Gew.% freie Fettsäuren.

### Beispiel 5

Ein Glyceridgemisch aus 58 Gew.% Monoglyceriden, 38 Gew.% Diglyceriden, 3,5 Gew.% Triglyceriden der Stearinsäure und 0,5 Gew.% freien Fettsäuren wurde bei einer Temperatur von 110°C und einem Druck von 85 bar einer Gegenstromkolonne 1 Meter oberhalb des Sumpfes zugepumpt und der Extraktion unterzogen. Dabei diente reines Propan als Extraktionsmittel. Seine Dichte beträgt unter den gegebenen Bedingungen 370 kg/m³. Die Kolonne war 6 m hoch und mit einer Sulzer-Drahtgewebepackung CY ausgerüstet. Die Beladung des Extraktionsmittels mit Glyceriden betrug 4 Gew.%.

Der die Trennkolonne am Kopf verlassende Extraktstrom wurde in die Regenerierkolonne überführt. Die Regenerierbedingungen waren 34,5 bar und 120°C. Die Dichte des Propans beträgt unter diesen Bedingungen 66 kg/m³. Durch die gleichzeitige Druckverminderung und Erwärmung nahm die Beladung des Extraktionsmittels auf 0,04 Gew.% ab.

Das so regenerierte Extraktionsmittel wurde auf 100 °C gekühlt und mit Hilfe eines Umlaufkompressors in die Extraktionskolonne am Boden zurückgeführt.

Ein Teil des in der Regenerierkolonne anfallenden Produktes wurde zum Kopf der Trennkolonne zurückgepumpt.

Das in der Regenerierkolonne anfallende Produkt enthielt 5 Gew.% Monoglyceride, 51,5 Gew.% Diglyceride, 42 Gew.% Triglyceride und 1,5 Gew.% freie Fettsäuren.

Das erhaltene Sumpfprodukt enthielt 62,8 Gew.% Monoglyceride, 36,8 Gew.% Diglyceride und 0,4 Gew.% freie Fettsäuren. Die Konzentration der Triglyceride lag im Bereich der Nachweisgrenze von 0,1 Gew.%.

Das Sumpfprodukt der ersten Kolonne wurde in einem zweiten Teilschritt bei einer Temperatur von 110°C und einem Druck von 85 bar einer zweiten Gegenstromkolonne in der Mitte zugepumpt und der Extraktion unterzogen. Dabei diente reines Propan als Extraktionsmittel. Die Kolonne war 12 m hoch und mit einer Sulzer-Drahtgewebepackung CY ausgerüstet. Die Beladung des Extraktionsmittels mit Glyceriden betrug 3,4 Gew.%.

Der die zweite Trennkolonne am Kopf verlassende Extraktstrom wurde in die zweite Regenerierkolonne überführt. Die Regenerierbedingungen waren 34,5 bar und 120°C. Durch die gleichzeitige Druckminderung und Erwärmung nahm die Beladung des Extraktionsmittels auf 0,04 Gew.% ab.

Das so regenerierte Extraktionsmittel wurde auf 100°C gekühlt und mit Hilfe eines zweiten Umlaufkompressors in die zweite Extraktionskolonne am Boden zurückgeführt.

Ein Teil des in der zweiten Regenerierkolonne anfallenden Produkts wurde zum Kopf der zweiten Trennkolonne zurückgeführt.

Das in der Regenerierkolonne anfallende Produkt enthielt 7,4 Gew.% Monoglyceride, 90 Gew.% Diglyceride und 0,6 Gew.% freie Fettsäuren. Die Konzentration der Triglyceride betrug 2 Gew.%.

Das erhaltene Sumpfprodukt enthielt 99 Gew.% Monoglyceride, 0,7 Gew.% Diglyceride und 0,3 Gew.% freie Fettsäuren. Die Konzentration der Triglyceride lag unterhalb der Nachweisgrenze von 0,1 Gew.%.

## Patentansprüche

1. Verfahren zur Gewinnung von Monoglyceriden und gegebenenfalls Diglyceriden und/oder Triglyceriden sowie gegebenenfalls Glycerin aus Glyceridgemischen bzw. glycerinhaltigen Glyceridgemischen durch Gegenstromextraktion mit einem im Kreislauf gehaltenen Extraktionsmittel, dadurch gekennzeichnet, daß als eine separate Phase bildendes Extraktionsmittel ein Kohlenwasserstoff mit einer Dichte von mehr als 180 kg/m³, vorzugsweise von 200 bis 800 kg/m³, und/oder Trifluormethan mit einer Dichte von mehr als 180 kg/m³, vorzugsweise von 300 bis 1100 kg/m³, verwendet wird.

2. Verfahren nach Anspruch 1, dadurch gekennzeichnet, daß als Extraktionsmittel ein Kohlenwasserstoff mit 2 bis 5 Kohlenstoffatomen, vorzugsweise Ethan, Propan, Butan, Pentan, Ethen, Propen, Buten, Penten oder deren Gemische verwendet werden.

3. Verfahren nach mindestens einem der Ansprüche 1 bis 2, dadurch gekennzeichnet, daß nach der Abscheidung des Sumpfproduktes in einer Trennsäule der die verbleibenden Glyceride enthaltende Extrakt durch schrittweise Dichteverminderung in mindestens einer weiteren folgenden Fraktionierkolonne in die einzelnen Glyceride aufgetrennt wird.

4. Verfahren nach mindestens einem der Ansprüche 1 bis 3, dadurch gekennzeichnet, daß die reinen Monoglyceride als Sumpfprodukt und der di- und triglyceridhaltige Extrakt am Kopf der Trennsäule abgeführt werden.

5. Verfahren nach mindestens einem der Ansprüche 1 bis 4, dadurch gekennzeichnet, daß nach erfolgter Abscheidung der Monoglyceride das Kopfprodukt der Trennsäule einer folgenden Fraktionierkolonne zugeführt, und die Diglyceride nach Dichteverminderung des Extraktionsmittels als Sumpfprodukt dieser Fraktionierkolonne abgeführt werden.

6. Verfahren nach mindestens einem der Ansprüche 1 bis 5, dadurch gekennzeichnet, daß nach erfolgter Abscheidung der Diglyceride in der ersten Fraktionierkolonne die Triglyceride aus dem Kopfprodukt der ersten Fraktionierkolonne durch weitere Dichteverminderung in einer zweiten Fraktionierkolonne abgeführt werden.

7. Verfahren nach mindestens einem der Ansprüche 1 bis 3, dadurch gekennzeichnet, daß in der Trennsäule Mono- und Diglyceride als Sumpfprodukt und der triglyceridhaltige Extrakt am Kopf der Trennsäule abgeführt werden.

8. Verfahren nach Anspruch 7, dadurch gekennzeichnet, daß das mono- und diglyceridhaltige Sumpfprodukt einer weiteren Trennsäule zugeführt wird.

9. Verfahren nach mindestens einem der Ansprüche 1 bis 6, dadurch gekennzeichnet, daß bei Anwesenheit von Glycerin in dem Glyceridgemisch in einem ersten Schritt das Glycerin und gegebenenfalls gleichzeitig die Monoglyceride abgetrennt werden.

10. Verfahren nach mindestens einem der Ansprüche 1 bis 9, dadurch gekennzeichnet, daß jeweils ein Teil der in der bzw. den Fraktionierkolonnen anfallenden Sumpfprodukte als Rücklauf auf den Kopf der vorhergehenden Kolonne gegeben wird.

11. Verfahren nach mindestens einem der Ansprüche 1 bis 10, dadurch gekennzeichnet, daß als Extraktionsmittel Ethan bei Temperaturen von 10 bis 120°C, vorzugsweise 20 bis 80°C, und Drücken von 50 bis 500 bar, vorzugsweise 100 bis 350 bar verwendet wird.

12. Verfahren nach mindestens einem der Ansprüche 1 bis 10, dadurch gekennzeichnet, daß als Extraktionsmittel Propan bei Temperaturen von 40 bis 150°C, vorzugsweise 60 bis 120°C und bei Drücken von 40 bis 350 bar, vorzugsweise von 70 bis 250 bar verwendet wird.

13. Verfahren nach mindestens einem der Ansprüche 1 bis 10, dadurch gekennzeichnet, daß als Extraktionsmittel ein Gemisch aus Ethan und Propan verwendet wird.

14. Verfahren nach mindestens einem der Ansprüche 1 bis 13, dadurch gekennzeichnet, daß die Gegenstromextraktion in einer gepackten Säule mit Druckpulsation durchgeführt wird.

15. Verfahren nach mindestens einem der Ansprüche 1 bis 14, dadurch gekennzeichnet, daß die Gegenstromextraktion in einer mit Siebböden ohne Ablaufschacht ausgerüsteten Kolonne durchgeführt wird und der Transport der in der Kolonne herablaufenden flüssigen Phase durch die Siebböden mit Hilfe von Druckpulsation erfolgt.

16. Verfahren nach mindestens einem der Ansprüche 1 bis 15, dadurch gekennzeichnet, daß die Gegenstromextraktion in einer rotierenden Scheibenkolonne durchgeführt wird.

17. Verfahren nach mindestens einem der Ansprüche 1-2, dadurch gekennzeichnet, daß aus glycerinhaltigen Glyceridgemischen Glycerin und Monoglyceride in einer ersten Gegenstromkolonne als Sumpfprodukt abgetrennt und die Di- und Triglyceride des Kopfprodukts nach Entspannung und Abtrennung des Extraktionsmittels zur weiteren Auftrennung in eine zweite Gegenstromkolonne mit separatem Kreislauf überführt werden, in der die Diglyceride als Sumpfprodukt und die Triglyceride als Kopfprodukt abgeführt werden.

18. Verfahren nach Anspruch 17, dadurch gekennzeichnet, daß ein Teil der aus den Kopfprodukten der Gegenstromkolonnen abgetrennten Produkte als Rücklauf auf den Kopf der jeweiligen Kolonne gegeben wird.

## Claims

1. A method for the production of monoglycerides and optionally diglycerides and/or triglycerides as well as optionally glycerol from glyceride mixtures and glycerol-containing glyceride mixtures, respectively, by countercurrent extraction with a circulating extractant,
characterized by using as an extractant forming a separate phase a hydrocarbon having a density of more than 180 kg/m³, preferably of from 200 to 800 kg/m³, and/or trifluoromethane having a density of more than 180 kg/m³, preferably of from 300 to 1100 kg/m³.

2. The method according to Claim 1, characterized by using as an extractant a hydrocarbon having 2 to 5 carbon atoms, preferably ethane, propane, butane, pentane, ethene, propene, butene, pentene, or mixtures thereof.

3. The method according to at least one of Claims 1 and 2, characterized in that, after separation of the bottom product in a separating column, the extract comprising the remaining glycerides is separated into the individual glycerides by stepwise reduction of density in at least one subsequent fractionating column.

4. The method according to at least one of Claims 1 to 3, characterized in that the pure monoglycerides are obtained as a bottom product, and the extract comprising di- and triglycerides is taken from the head of the column.

5. The method according to at least one of Claims 1 to 4, characterized in that, after separation of the monoglycerides, the head product from the separating column is fed into a subsequent fractionating column, and the diglycerides, after density reduction of the extractant, are withdrawn as the bottom product of said fractionating column.

6. The method according to at least one of Claims 1 to 5, characterized in that, after completed separation of the diglycerides in a first fractionating column, the triglycerides from the head product of said first fractionating column are recovered after further density reduction in a second fractionating column.

7. The method according to at least one of Claims 1 to 3, characterized in that the mono- and diglycerides in the separating column are drawn off as a bottom product, and the extract comprising triglycerides is taken from the head of said separating column.

8. The method according to Claim 7, characterized in that the bottom product comprising mono- and diglycerides is fed into a further separating column.

9. The method according to at least one of Claims 1 to 6, characterized in that, when glycerol is present in the glyceride mixture, said glycerol and optionally the monoglycerides, too, are separated in a first step.

10. The method according to at least one of Claims 1 to 9, characterized in that part of the bottom product obtained in the fractionating column(s) is recycled as return to the head of the preceding column.

11. The method according to at least one of Claims 1 to 10, characterized by using as an extractant ethane at temperatures of from 10 to 120°C, preferably of from 20 to 80°C, and pressures of from 50 to 500 bar, preferably of from 100 to 350 bar.

12. The method according to at least one of Claims 1 to 10, characterized by using as an extractant propane at temperatures of from 40 to 150°C, preferably of from 60 to 120°C, and pressures of from 40 to 350 bar, preferably of from 70 to 250 bar.

13. The method according to at least one of Claims 1 to 10, characterized by using as an extractant a mixture of ethane and propane.

14. The method according to at least one of Claims 1 to 13, characterized in that countercurrent extraction takes place in a packed column with the aid of pulsating pressure.

15. The method according to at least one of Claims 1 to 14, characterized in that countercurrent extraction takes place in a column provided with sieve plates without discharge shaft, and that the liquid phase flowing downwards in the column is transported through said sieve plates with the aid of pulsating pressure.

16. The method according to at least one of Claims 1 to 15, characterized in that countercurrent extraction is conducted in a rotary-disk column.

17. The method according to at least one of Claims 1 and 2, characterized in that glycerol and monoglycerides from a glycerol-containing mixture of glycerides are separated in a first countercurrent column as a bottom product, and that the di- and triglycerides of the head product are fed after expansion and separation of the extractant into a second countercurrent column (separate circulation system) for further separation, from which the diglycerides are taken as a bottom product and the triglycerides as a head product.

18. The method according to Claim 17, characterized in that part of the products separated from the head products of the countercurrent columns are recycled as return to the head of the respective column.

## Revendications

1. Procédé pour l'obtention de monoglycérides et éventuellement de diglycérides et/ou de triglycérides ainsi qu'éventuellement de glycérine à partir de mélanges de glycérides ou de mélanges de glycérides contenant de la glycérine par extraction à contre-courant avec un agent d'extraction maintenu en recyclage, caractérisé en ce qu'un hydrocarbure d'une masse volumique supérieure à 180 kg/m³, de préférence de 200 à 800 kg/m³ et/ou du trifluorométhane d'une masse volumique supérieure à 180 kg/m³, de préférence de 300 à 1100 kg/m³, est utilisé comme agent d'extraction formant une phase séparée.

2. Procédé selon la revendication 1, caractérisé en ce qu'un hydrocarbure avec 2 à 5 atomes de carbone, de préférence l'éthane, le propane, le butane, le pentane, l'éthène, le propène, le butène, le pentène ou leurs mélanges, sont employés comme agents d'extraction.

3. Procédé selon au moins une des revendications 1 à 2, caractérisé en ce que, après la séparation du produit de pied de colonne dans une colonne de séparation, l'extrait contenant les glycérides restants est séparé en glycérides individuels par un abaissement progressif de la densité dans au moins une autre colonne de fractionnement lui faisant suite.

4. Procédé selon au moins une des revendications 1 à 3, caractérisé en ce que les monoglycérides purs sont soutirés sous la forme de produit de pied de colonne et que l'extrait contenant les di- et triglycérides est soutiré en tête de la colonne de séparation.

5. Procédé selon au moins une des revendications 1 à 4, caractérisé en ce que, après la séparation des monoglycérides, le produit de tête de la colonne de séparation est envoyé dans une colonne de fractionnement lui faisant suite et que les diglycérides, après abaissement de la densité de l'agent d'extraction, sont soutirés de cette colonne de fractionnement sous la forme de produit de pied de colonne.

6. Procédé selon au moins une des revendications 1 à 5, caractérisé en ce que, après la séparation effectuée des diglycérides dans la première colonne de fractionnement, les triglycérides sont soutirés du produit de tête de la première colonne de fractionnement par un nouvel abaissement de densité dans une deuxième colonne de fractionnement.

7. Procédé selon au moins une des revendications 1 à 3, caractérisé en ce que, dans la colonne de séparation, les mono- et diglycérides sont soutirés sous la forme de produit de pied de colonne et que l'extrait contenant les triglycérides est soutiré en tête de la colonne de séparation.

8. Procédé selon la revendication 7, caractérisé en ce que le produit de pied de colonne contenant les mono- et diglycérides est envoyé dans une autre colonne de séparation.

9. Procédé selon au moins une des revendications 1 à 6, caractérisé en ce que, en présence de glycérine dans le mélange de glycérides, la glycérine et éventuellement aussi les monoglycérides sont séparés dans une première étape.

10. Procédé selon au moins une des revendications 1 à 9, caractérisé en ce que chaque fois une partie des produits de pied de colonne obtenus dans la ou les colonnes de fractionnement est chargée comme reflux en tête de la colonne précédente.

11. Procédé selon au moins une des revendications 1 à 10, caractérisé en ce que de l'éthane est employé comme agent d'extraction à des températures de 10 à 120°C, de préférence de 20 à 80°C et sous des pressions de 50 à 500 bars, de préférence de 100 à 350 bars.

12. Procédé selon au moins une des revendications 1 à 10, caractérisé en ce que du propane est employé comme agent d'extraction à des températures de 40 à 150°C, de préférence de 60 à 120°C et sous des pressions de 40 à 350 bars, de préférence de 70 à 250 bars.

13. Procédé selon au moins une des revendications 1 à 10, caractérisé en ce qu'un mélange d'éthane et de propane est employé comme agent d'extraction.

14. Procédé selon au moins une des revendications 1 à 13, caractérisé en ce que l'extraction à contre-courant s'effectue dans une colonne à garnissage pulsée.

15. Procédé selon au moins une des revendications 1 à 14, caractérisé en ce que l'extraction à contre-courant est conduite dans une colonne équipée de plateaux perforés sans déversoir et que le transport de la phase liquide s'écoulant vers le bas de la colonne a lieu à travers les plateaux perforés à l'aide d'une pulsation sous pression.

16. Procédé selon au moins une des revendications 1 à 15, caractérisé en ce que l'extraction à contre-courant s'effectue dans une colonne à disques rotative.

17. Procédé selon au moins une des revendications 1 à 2, caractérisé en ce que, à partir de mélanges de glycérides contenant de la glycérine, la glycérine et les monoglycérides sont séparés sous la forme de produit de pied de colonne dans une première colonne à contre-courant et qu'après détente et séparation de l'agent d'extraction, les di- et triglycérides du produit de tête sont transférés, en vue d'une séparation ultérieure, dans une deuxième colonne à contre-courant avec circuit séparé dans laquelle les diglycérides sont soutirés sous la forme de produit de pied de colonne et les triglycérides sous la forme de produit de tête.

18. Procédé selon la revendication 17, caractérisé en ce qu'une partie des produits séparés des produits de tête des colonnes à contre-courant est chargée comme reflux en tête de la colonne correspondante.
